# EUROPÄISCHE PATENTSCHRIFT

(11) **EP 1 501 966 B1**
(45) Veröffentlichungstag und Bekanntmachung des Hinweises auf die Patenterteilung: **07.05.2014**
(21) Anmeldenummer: 03712127.4
(22) Anmeldetag: 02.04.2003
(51) Int. Cl.: D01H 5/32, D01H 5/38, D01H 5/42, G01N 22/00

(54) **SPINNEREIVORBEREITUNGSMASCHINE MIT MIKROWELLENSENSOREN**
SPINNING PREPARATION MACHINE WITH MICROWAVE SENSORS
MACHINE DE PREPARATION DE FILATURE AVEC CAPTEURS A MICRO-ONDES

(30) Priorität: 04.04.2002 DE 10214955
(43) Veröffentlichungstag der Anmeldung: 02.02.2005
(60) Teilanmeldung: 11170772.5
(73) Patentinhaber: Rieter Ingolstadt GmbH, 85055 Ingolstadt (DE)
(72) Erfinder: DÄMMIG, Joachim, 85053 Ingolstadt (DE); UEDING, Michael, 85049 Ingolstadt (DE); CHERIF, Chokri, 85057 Ingolstadt (DE)
(74) Vertreter: Schlief, Thomas P.
(86) Internationale Anmeldenummer: PCT/EP2003/003442
(87) Internationale Veröffentlichungsnummer: WO 2003/085179

(56) Entgegenhaltungen:
- EP-A- 0 678 601
- EP-A- 0 692 560
- EP-A- 1 316 630
- WO-A-00/12974
- DE-A- 10 204 328
- US-A- 4 653 153
- US-A- 4 809 405
- US-A- 4 864 853
- US-A- 4 949 431
- US-A- 5 697 247
- US-A- 5 815 890
- US-A- 5 943 740
- US-A- 6 088 882

## Beschreibung

Die Erfindung betrifft eine Spinnereivorbereitungsmaschine.

In der Spinnereiindustrie wird beispielsweise aus Baumwolle in mehreren Prozeßschritten zuerst ein vergleichmäßigter Faserverband und schließlich als Endprodukt ein gedrehtes Garn produziert. Die der Garnherstellung vorgeordneten Spinnereivorbereitungsmaschinen, wie Karden, Kämmmaschinen und Strecken, haben insbesondere die Aufgabe, die Bandmasseschwankungen eines oder mehrerer Faserbänder auszuregulieren. Zu diesem Zweck sind beispielsweise an Strecken Bandsensoren angeordnet, welche die Banddicke bzw. den Bandquerschnitt bzw. die Bandmasse bzw. deren Schwankungen messen und diese Informationen an eine Reguliereinheit weitergeben, die mindestens eines der Verzugsorgane des Streckwerks entsprechend ansteuert. Eine nach einem solchen Regulierprinzip arbeitende Strecke ist beispielsweise das Modell RSB-D 30 der Firma RIETER. Auch bei unregulierten Strecken sind Informationen hinsichtlich der Banddickeschwankungen in vielen Fällen erwünscht. Ein entsprechender Sensor am Auslauf einer solchen Strecke gibt beispielsweise ein entsprechendes Abschaltsignal für die Maschine und/oder ein Warnsignal aus, wenn ein Schwellenwert der Bandmasse bzw. der Banddicke unter- bzw. überschritten wird.

Zur Messung der Banddickenschwankungen sind insbesondere mechanische Abtastungen bekannt, die sich heutzutage in fast allen entsprechenden Maschinen durchgesetzt haben. Allerdings reicht die Dynamik dieser mechanischen Sensoren bei Liefergeschwindigkeiten von mehr als 1000 m/min bei einem hohen Anforderungsprofil nicht mehr in genügendem Maße aus. Zudem macht sich die notwendige starke mechanische Verdichtung vor dem mechanischen Sensor negativ auf die Verzugsfähigkeit bemerkbar.

Neben der mechanischen Abtastung der Banddickenschwankungen sind weitere Abtastprinzipien vorgeschlagen worden. So ist beispielsweise aus der US 2,942,303 sowie der DE 44 45 720 A1 bekannt, die Banddicke berührungslos mit durchdringender optischer Strahlung zu messen. Jedoch wird die Meßgenauigkeit hierbei stark von den Umgebungseinflüssen, z.B. Temperatur, Feuchtigkeit und Schmutz, beeinflußt. Außerdem ist das Verfahren anfällig gegenüber Farbe sowie Reflektionseigenschaften des Faserverbandes.

Bei weiteren bekannten berührungslos arbeitenden Meßverfahren werden Ultraschallwellen verwendet. Ebenfalls bekannt sind kapazitiv oder pneumatisch arbeitende Meßmethoden. Auch ist vorgeschlagen worden, Röntgenstrahlung oder gamma-Strahlen zu verwenden. Allen diesen Verfahren ist jedoch gemeinsam, daß sie feuchteempfindlich sind. Es nützt daher wenig, daß klimatische Einflüsse wie die Temperatur und die relative Luftfeuchtigkeit sich in der Regel kompensieren lassen, um klimatische Einflüsse minimieren zu können. Das Problem der inhärenten Faserfeuchte läßt sich hierdurch nicht ohne weiteres beseitigen. Zudem kann bei ein und derselben Baumwollpartie die Faserfeuchte bei gleichbleibenden Umgebungsbedingungen bis zu 5 % variieren. Auch nehmen die oberen Baumwolllagen in einer Kanne, die einer Spinnereivorbereitungsmaschine zugestellt wird, mehr Feuchtigkeit auf als die darunter liegenden. Außerdem weisen die textilen Fasern durch die Veränderung der klimatischen Bedingungen innerhalb einer Spinnerei - z.B. morgens vs. mittags vs. abends - unterschiedliche Feuchte auf. Die genannten Einflüsse haben ihrerseits einen großen Einfluß auf das Meßergebnis der Banddicke und somit auf die Regulierungsgüte. Insgesamt sind diese Verfahren also kaum für hochpräzise Messungen der Faserbanddicke geeignet.

Eine relativ neue Methode zur Messung der Banddicke beruht auf der Verwendung von Mikrowellen. In der WO 00/12974 ist ein derartiges Meßverfahren unter Verwendung von Mikrowellen beschrieben, bei der Mikrowellen- in einen Resonator eingekoppelt werden, durch die ein oder mehrere Faserbänder geführt werden. Es wird dann die Dämpfung und die Resonanzfrequenzverschiebung aufgrund der Anwesenheit des oder der Faserbänder gemessen und aus den Meßwerten auf die Dickenschwankungen und evtl. den Feuchtegehalt des oder der Faserbänder geschlossen. In der EP 0 468 023 B1 ist ein ähnliches Mikrowellen-Meßverfahren beschrieben, das sich auf die Messung von Faserbändern übertragen läßt. Die auf der Mikrowellen-Resonatortechnik beruhenden Sensoren bieten insbesondere den Vorteil, daß die Umweltbedingungen - wie beispielsweise die Raumtemperatur und die Raumfeuchte - schon mitberücksichtigt sind, so daß diese nicht weiter kompensiert werden müssen.

US-A-6,088,882 zeigt eine Maschine nach dem Oberbegriff von Anspruch 1.

Allerdings sind die in den genannten Druckschriften beschriebenen und dargestellten Sensoren sowie die entsprechenden Meßverfahren in vielfacher Hinsicht unausgereift und verbesserungswürdig. Insbesondere die spezifische Anpassung an die Problematik bei der Vermessung von Faserbändern erfordert neue Lösungen.

Es ist Aufgabe der Erfindung, die Anpassung der Mikrowellensensoren bei der Vermessung von Faserbändern bzw. eines Faserverbandes zu verbessern.

Diese Aufgabe wird bei einer Spinnereivorbereitungsmaschine gemäß dem unabhängigen Anspruch 1 gelöst.

Erfindungsgemäß ist vorgesehen, einen Mikrowellensensor zwischen einer dem Streckwerk nachgeschalteten Vliesdüse und einem weiter stromabwärts angeordneten Kalanderwalzenpaar vorzusehen.

Bei Verschmutzungen des Mikrowellenresonators ist zwischen zwei Arten von Verschmutzungen zu unterscheiden. Zum einen sind dies leicht entfernbare Verschmutzungen, wie beispielsweise Faserflug, zum anderen schwer entfernbare Verschmutzungen, wie beispielsweise Honigtau und Avivage. Diese beiden Verschmutzungen führen zur Veränderung der Resonator-Kennwerte, so daß eine Reinigung des oder der Resonatoren vorgeschlagen wird.

Eine Schmutzentfernung kann in regelmäßigen Abständen stattfinden, beispielsweise und bevorzugt im Stoppzustand der Maschine. Die entsprechenden Reinigungseinrichtungen zur Reinigung des oder der Mikrowellenresonatoren von leicht oder schwer zu entfernenden Verschmutzungen können bei Überschreiten von vordefinierten Grenzwerten, z.B. hinsichtlich der Resonator-Kennwerte im leeren Zustand des Resonators, oder bei Überschreiten von Schmutz- oder Schmierfilmdicken, mittels geeigneter Steuermittel ausgelöst und/oder die Notwendigkeit einer Reinigung angezeigt werden. Die Reinigung kann entweder manuell oder mit Hilfe einer Reinigungseinrichtung durchgeführt werden, wobei die manuelle Reinigung bei den schwer zu entfernenden Verschmutzungen in einigen Fällen unabdingbar sein kann.

Die leichter entfernbaren Verschmutzungen lassen sich vorzugsweise durch Druckluft entfernen, wobei ein oder mehrere Luftdüsen auf den Meßschlitz des Resonators gerichtet sind.

Vorzugsweise sind Steuerungsmittel vorhanden, welche bei schwer oder nicht zu entfernenden Verschmutzungen ein Stoppen der Maschine bedingen. Bevorzugt wird jedoch aus Gründen der Produktivität die Reinigung - sowohl der leichter als auch der schwerer zu entfernenden Verschmutzungen - bei einem Kannenwechsel am Streckwerksauslauf oder einem Vorlagekannenwechsel vorgenommen, da dann die Maschine in der Regel kein Faserband produziert (außer bei einem sog. fliegenden Wechsel). Die Steuerungsmittel können in einem zentralen Maschinenrechner integriert sein.

Für die Reinigung ist der Mikrowellensensor bevorzugt ausfahrbar ausgebildet, beispielsweise mittels eines Motors und einer Laufschiene, auf der der Sensor verfahrbar ist, wobei das Faserbandmaterial bevorzugt in seiner Lage unverändert bleibt und vorzugsweise in dieser Position mittels geeigneter Haltemittel fixiert ist. Die Reinigung des Sensors erfolgt bevorzugt mittels Druckluft oder mechanischer Reinigungsmittel, welche die Resonatorauskleidung - z.B. Keramik - schonen. Bei einem stationär ausgebildeten Sensor sind die Verschmutzungen manuell oder automatisch (mittels Druckluft, mechanischer Reinigungsmittel etc.) aus dem Meßschlitz zu entfernen. Nach der Reinigung mit beispielsweise Druckluft kann vorzugsweise mittels einer elektronischen Auswerteeinheit durch Auswertung der Resonator-Kennwerte im leeren Zustand (Güte) festgestellt werden, ob Schmutz noch anhaftet oder nicht, wobei die Grenzwerte für schwer entfernbares Material zu beachten sind.

Die Steuerungsmittel zur Steuerung der Sensorenreinigung können in einem zentralen Maschinenrechner integriert sein.

Um den Verschmutzungsgrad des Resonators zu minimieren, wird der Meßraum vorzugsweise derart konstruktiv gestaltet, daß eine Anhaftung von Verschmutzungen vermindert oder sogar verhindert wird. Es bietet sich hierbei vorzugsweise an, die Innenoberfläche des Sensors aus schmutzabweisenden und abriebfesten Werkstoffen zu gestalten und/oder scharfe Kanten zu vermeiden, insbesondere an den Ein- und Auslaufstellen des Faserbandmaterials in den Sensor.

Bevorzugt weist die Spinnereivorbereitungsmaschine Einfädelmittel zur automatischen Einfädelung des Faserbandmaterials in den mindestens einen Sensor bei der Verarbeitung von neuen Partien bzw. Behebung von Bandbrüchen auf. Derartige Einfädelmittel umfassen beispielsweise eine oder mehrere Luftdüsen, so daß das Faserbandmaterial von dem erzeugten Luftstrom erfaßt und in den Sensor eingeführt wird. Alternativ oder zusätzlich können die Einfädelmittel auch auf mechanischer Basis arbeiten, beispielsweise durch Klemmung und Bewegung bzw. Einführung des oder der Faserbänder in den Meßschlitz des Resonators.

Die Einfädelmittel können weiterhin mechanisch wirkende Haltemittel- beispielsweise Klemmen - umfassen, mit denen bei Reinigungsarbeiten (s.o.) das Faserbandmaterial nach Ausfahren des Sensors aus einer Meßposition in eine Reinigungsposition in einer definierten Position gehalten werden kann. Auf diese Weise kann anschließend das Material ohne manuellen Eingriff in den Meßschlitz des wieder in Meßposition verfahrenen Sensors eingeführt werden.

Weiterhin wurde erkannt, daß das zu verarbeitende Fasermaterial am Einlauf und Auslauf der Strecke unterschiedliche Temperaturverläufe aufweisen kann, welche die Meßergebnisse verfälschen können. Diesbezüglich wird vorgeschlagen, daß die Spinnereivorbereitungsmaschine eine Einrichtung zur Temperaturmessung aufweist, um die Temperaturen des textilen Fasermaterials mit mindestens jeweils einem Temperaturfühler bevorzugt fortlaufend (inklusive Start/Stoppphase und insbesondere Kaltstart) zu ermitteln und damit die Meßergebnisse zu kompensieren. Dies kann entsprechend einer vorteilhaften Ausführungsform in der Meßelektronik des Mikrowellensensors geschehen oder durch externe Kompensation. Auf diese Weise können insbesondere Kreuzkorrelationen der Meßergebnisse am Einlauf und Auslauf vorgenommen werden, wobei vorzugsweise die unterschiedlichen Bandgeschwindigkeiten am Ein- und Auslauf sowie die Laufzeit zwischen den beiden Sensoren berücksichtigt werden, z.B. beispielsweise in einem zentralen Rechner der Maschine. Je nach Temperaturdifferenz zwischen Material am Einlauf und Auslauf soll eine Korrektur vorgenommen werden (Offset-Korrektur).

Bei der Verwendung von Sensoren auf Mikrowellenbasis ist die elektrische Leitfähigkeit von Mattierungselementen und Pigmenten in zu verstreckender oder verstreckter Baumwolle in den meisten Fällen unbedenklich. Bei elektrisch leitenden Werkstoffen, wie z.B. Kohlefasern, kann ggf. derselbe Mikrowellensensor eingesetzt werden. Gleichfalls kann ein zweiter Sensor verwendet werden, der vorzugeweise nach einem anderen physikalischen Prinzip arbeitet.

Werden jeweils mindestens zwei Resonatoren an einer Meßposition - d.h. entweder am Einlauf oder am Auslauf - hintereinander geschaltet, kann mit ihnen bevorzugt ein Bandfilter realisiert werden.

Gemäß einem weiteren Aspekt ist mindestens ein Mikrowellensensor zur Messung der Faserbanddicke am Einlauf und mindestens ein Mikrowellensensor zur Messung der Faserbanddicke am Auslauf vorgesehen, wobei die Sollbandfeinheit des die Maschine verlassenden Faserbandes vorgebbar ist, beispielsweise über ein Maschinendisplay. Die Maschine ist derart ausgebildet, daß die vom mindestens einen Sensor am Einlauf und dem mindestens einen Sensor am Auslauf gemessenen Ist-Bandfeinheiten mit Hilfe einer Auswerteeinheit, die beispielsweise in einem zentralen Maschinenrechner integriert ist, miteinander korrelierbar und die Ergebnisse an eine Steuereinheit weitergebbar sind, um die Verzugsorgane gemäß der vorgegebenen Sollbandfeinheit entsprechend anzusteuern. Vorzugsweise wird mittels der Auswerteeinheit eine Kreuzkorrelation zwischen den von dem mindestens einen Sensor am Einlauf und dem mindestens einen Sensor am Auslauf gemessenen Ist-Bandfeinheiten vorgenommen. Eine anschließende Plausbilitätskontrolle ist vorteilhaft.

Zur Kalibrierung des mindestens einen Mikrowellensensors werden bevorzugt Kalibrierungskurven für unterschiedliche Materialien erstellt, wobei diese Kurven in der Meßelektronik speicherbar sind und/oder ein Abrufen der Kurven bei Bedarf von externen elektronischen Medien erfolgen kann, z.B. über das Internet, eine Compact Disc etc..

Für jedes Fasermaterial, z.B. Baumwolle, Polyester, Viskose, Polyacrylnitril etc, wird bevorzugt mindestens eine Kalibrierungskurve erstellt. Insbesondere in Abhängigkeit von beispielsweise Kräuselungsgrad, Feuchteaufnahmevermögen, Vorbehandlungsgrad, Verschmutzungsgrad etc. können auch vorteilhafterweise mehrere Kalibrierungskurven aufgenommen werden.

Wenn Fasermischungen vorliegen - z.B. Flockenmischungen und/oder Bandmischungen - sind neue Kalibrierungskurven in Abhängigkeit vom Mischungsverhältnis zu bestimmen. Diese Kurven können z.B. in einem elektronischen Speicher hinterlegt sein oder aufgrund einer Eingabe des Mischungsverhältnisses aus den entsprechenden einzelnen Kalibrierungskurven berechnet bzw. bestimmt werden. Hierzu kommen insbesondere mathematische Operationen in Frage, z.B. Mittelungen, Interpolationen oder Regressionen. Alternativ oder zusätzlich sind diese Daten für Mischungsverhältnisse in einem elektronischen Speicher hinterlegt oder können aufgrund der obigen Berechnungen in einen solchen Speicher geschrieben werden. Auf diese Weise steht dem Anwender eine Datenbank für die verschiedenen Mischungskombinationen zur Verfügung, die er bei der jeweiligen, gerade zu verstreckenden Partie abrufen kann.

Zur Eingabe der Mischungsverhältnisse weist die Spinnereivorbereitungsmaschine zweckmäßigerweise eine entsprechend ausgebildete Eingabeeinheit sowie eine Prozessoreinheit zur Bestimmung der Kalibrierungskurven aus den eingegebenen Mischungsverhältnissen auf.

Als Kalibrierungsmittel werden bevorzugt textile Faserbänder in Strangform mit definiertem Feuchtegehalt verwendet. Hierzu bieten sich beispielsweise konditionierte Proben an, bei denen die Faserfeuchte genau bekannt ist. Alternativ wird das gesamte Fasermaterial unter gleichen Raumbedingungen gelagert. Hierbei wird als Kalibrierungsmittel ein Teil dieses Fasermaterials verwendet. Es ist auch möglich, daß diese beiden Vorgehensweisen kombiniert werden.

Alternativ wird das zu verstreckende Material bei normalen Produktionsbedingungen in definierter Länge gewogen, anschließend getrocknet und in diesem Zustand wieder gewogen. Aus einem Vergleich dieser Bandfeinheiten wird der Feuchtegehalt bestimmt. Die Bandfeinheiten und der errechnete Feuchtegehalt werden anschließend der Auswerteeinheit des Mikrowellensensors zur Verfügung gestellt. Es können auch Bandmassen statt Bandfeinheiten verarbeitet werden. Die Kalibrierungskurve wird aus den gewogenen Bandfeinheiten und den dazugehörigen ermittelten Resonatorkennwerten, d.h. Frequenzverschiebung A und Feuchtigkeitsgehalt Φ, bestimmt. Die im wesentlichen lineare, in der Regel durch den Nullpunkt verlaufende Funktion "Frequenzverschiebung aufgetragen gegen Bandfeinheit" wird hierbei bei normalen Produktionsbedingungen mit dem Mikrowellensensor gemessen und der aus der Wägung errechneten Faserfeuchte zugeordnet. Vorteilhafterweise wird mindestens ein zweiter Meßpunkt von dem gleichen Material mit einem unterschiedlichen Feuchtegehalt ermittelt. Hiermit können unterschiedliche Feuchtegehalte während des Produktionlaufs ermittelt werden.

Bei einer weitergehenden Kalibrierung wird der Mikrowellen-Ausgangssensor aufgrund von Labormessungen nachkalibriert, bei denen beispielsweise die Ist-Bandfeinheit (und/oder die Bandfeuchtigkeit) des verstreckten Faserbandes gemessen wird (Plausibilitätskontrolle). Auf Basis dieser Nachkalibrierung, d.h. der aktuellen Kennlinie des Ausgangssensors, wird der Mikrowellen-Eingangssensor vorteilhafterweise unter besonderer Berücksichtigung der unterschiedlichen Faserband-Temperaturen am Ein- und Auslauf und weiteren Einflüssen, z.B. Verschmutzungsgrad des Sensors, nachkalibriert. Dies kann beispielsweise dann von Vorteil sein, wenn das einlaufende und das auslaufende Faserbandmaterial unterschiedliche Temperaturen aufweisen, welche die Meßergebnisse beeinflussen. Die Nachkalibrierung wird bevorzugt automatisch mit Hilfe eines Mikroprozessors vorgenommen.

Für die schnelle Kalibrierung des Mikrowellensensore werden vorzugsweise definierte textile Proben in Polymerverbindungen mit bekannten Massen eingegossen. Alternativ werden Ausgangspolymere der betreffenden Faserstoffe (Filamentgarne) verwendet, beispielsweise Viskoseschmelzen. Die Proben weisen vorzugsweise einen unterschiedlichen, bekannten Feuchtegehalt auf.

Vorteilhafterweise werden Proben zur Kalibrierung des mindestens einen Mikrowellensensors herangezogen, welche eine nahezu gleiche Dielektrizitätskonstante wie das zu verarbeitende Faserbandmaterial aufweisen.

Vorzugsweise wird eine einzige Auswerteelektronik für alle Resonatoren am Streckwerkseinlauf und/oder Streckwerksauslauf eingesetzt.

Die Erfindung läßt sich bei Karden, Strecken sowie Kämmmaschinen mit reguliertem als auch unreguliertem Streckwerk einsetzen. Auch ist ein Einsatz der Erfindung in einer Kombination aus einer Karde und nachgeschaltetem Streckwerk vorteilhaft.

Vorteilhafte Weiterbildungen der Erfindung sind durch die Merkmale der Unteransprüche gekennzeichnet.

Im folgenden wird die Erfindung in ihren verschiedenen Aspekten anhand der Figuren näher erläutert. Es zeigen:
- Figur 1: eine Regulierstrecke mit Regulierungskomponenten in schematischer Darstellung, und
- Figur 2: eine perspektivische Ansicht eines offenen Mikrowellensensors.

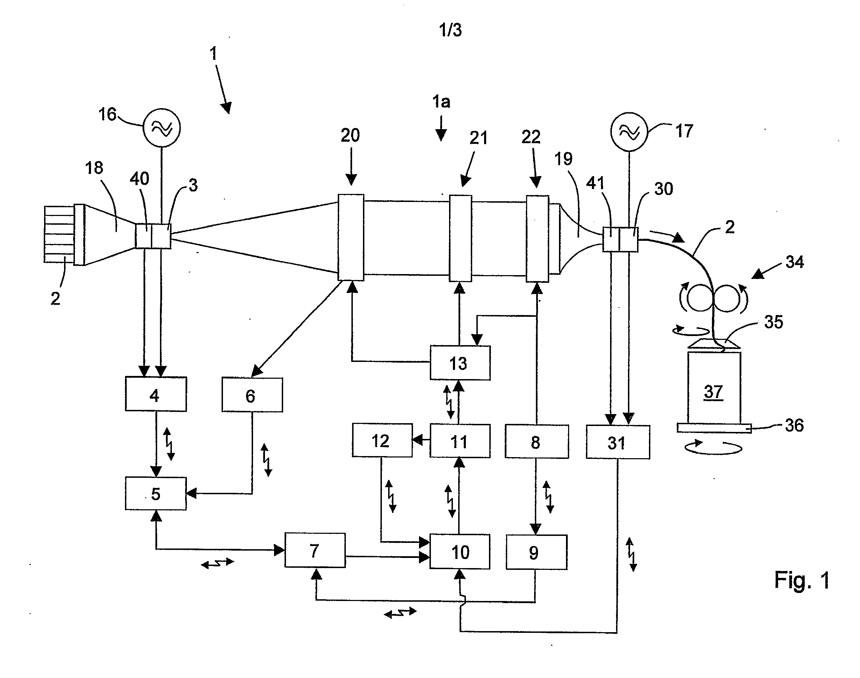

In Figur 1 ist schematisch ein beispielhaftes Regulierungsprinzip an einer Strecke 1 dargestellt. Am Eingang der Strecke 1 wird die Banddicke von einlaufenden Faserbänder 2 - in diesem Fall sechs Faserbänder 2 - mit einem Mikrowellensensor 3, der nach dem Resonatorprinzip arbeitet, erfaßt. Der Sensor 3 ist mit einem Mikrowellengenerator 16 verbunden, der von einer (nicht dargestellten) Prozessoreinheit angesteuert wird und Mikrowellen in den Resonator des Sensors 3 einkoppelt. Dem Sensor 3 ist ein als Verdichtungsmittel ausgebildeter Trichter 18 zum Verdichten der Faserbänder 2 vorgeschaltet. Nach Passieren des Mikrowellensensors 3 werden die Faserbänder 2 wieder ausgebreitet, um in das Streckwerk 1a einzulaufen. Die Meßwerte des Sensors 3 werden von einer Auswerteeinheit 4 in die Banddickenschwankungen repräsentierende elektrische Spannungswerte umgewandelt, die einem Speicher 5 zugeführt werden (elektrische Signale sind in der Figur 1 mit einem gezackten Doppelpfeil gekennzeichnet, während mechanische Signale keine spezielle Kennzeichnung aufweisen). Der Speicher 5 gibt die Meßspannung mit Unterstützung eines Impulsgebers bzw. Taktgebers 6 mit definierter zeitlicher Verzögerung an eine Sollwertstufe 7 weiter. Der Impulsgeber 6 erhält von einem Eingangswalzenpaar 20, das gleichzeitig zur Förderung der Faserbänder 2 durch den Sensor 3 dient, entsprechend der Bandlängenförderung durch dieses Walzenpaar 20 ein Trigger-Signal (sog. "vordefinierte konstante Abtastlänge"). Alternativ kann der Impulsgeber mit einem anderen Walzenpaar gekoppelt sein, beispielsweise mit einem (nicht dargestellten) Transportwalzenpaar unmittelbar hinter dem Sensor 3 (in Bandlaufrichtung gesehen). In einem solchen Fall dient nicht das Eingangswalzenpaar 20 zum Transport der Faserbänder 2 durch den Sensor 3, sondern das Transportwalzenpaar.

Die Sollwertstufe 7 erhält außerdem von einem Leittacho 9 eine Leitspannung, die ein Maß für die Drehzahl der unteren, von einem Hauptmotor 8 angetriebenen Walze eines Lieferwalzenpaares 22 ist. Anschließend wird in der Sollwertstufe 7 eine Sollspannung errechnet und an eine Steuereinheit 10 weitergegeben. In der Steuereinheit 10 findet ein Soll-Istwert-Vergleich statt, der dazu benutzt wird einem Regelmotor 11 eine ganz bestimmte, der gewünschten Verzugsänderung entsprechende Drehzahl zu erteilen, Die Istwerte des Regelmotors 11 werden hierbei einem Istwert-Tacho 12 übermittelt, der die entsprechende Ist-Spannung dann an die Steuereinheit 10 weitergibt. Der Regelmotor 11 treibt in ein vom Hauptmotor 8 angetriebenes Planetengetriebe 13, das die Drehzahlen der unteren Walze des Eingangswalzenpaares 20 und der unteren Walze 21 eines Mittelwalzenpaares 21 derart verändert, daß eine Bandvergleichmäßigung bzw. -verstreckung stattfindet.

Als Regelgröße dient die Banddicke. Aufgrund des Faserbandtransports vom Sensor 3 zum Verzugsfeld - bestehend aus dem Eingangs-, Mittel- und Lieferwalzenpaar 20, 21, 22 (die Walzenpaare sind in der Aufsicht dargestellt) - wird eine Totzeit errechnet. Die Drehzahl für den Regelmotor 11 als Stellgröße wird von der Steuereinheit 10 ermittelt, wobei hierbei die Ist-Dicke der Faserbänder 2, der Banddickensollwert (als Führungsgröße) und die Drehzahlen des Hauptmotors 8 und des Regelmotors 11, verarbeitet werden. Durch die proportionale Überlagerung der Drehzahlen des Hauptmotors 8 und des Regelmotors 11 und unter Berücksichtigung der genannten Totzeit wird die Banddicke im Streckwerk 1a im sog. Regeleinsatzpunkt geregelt.

Am Auslauf des Streckwerks a ist ein mit einem weiteren Mikrowellengenerator 17 verbundener Resonator eines Mikrowellensensors 30 angeordnet, der erfindungsgemäß einem als Verdichtungsmittel ausgebildeten Bandtrichter bzw. einer Vliesdüse 19 nachgeschaltet ist. Die Signale des Sensors 30 werden einer Auswerteeinheit 31 zugeführt, die elektrische Spannungssignale entsprechende der Banddicke des verstreckten Faserbandes 2 liefert und an die Steuereinheit 10 weitergibt. Alternativ oder zusätzlich dienen die Ergebnisse vom Sensor 30 lediglich zur Bandüberwachung und zur Bandqualitätskontrolle. Entsprechend werden diese Ergebnisse bevorzugt auf einem Display an der Maschine und/oder an einer zentralen Einheit in der Spinnerei angezeigt.

Andere Schaltungsanordnungen sind möglich, insbesondere der Einsatz eines zentralen Rechners.

Das verstreckte Faserband 2 wird mittels Kalanderwalzen 34 aus dem Mikrowellensensor 30 abgezogen (die Distanzen zwischen Sensor 30 und Walzen 34 sind nicht maßstäblich) und mit Hilfe eines in einem rotierenden Drehteller 35 angeordneten Bandkanals in eine Rundkanne 37 abgelegt, welche auf einem rotierenden Kannenteller 36 steht. Alternativ ist die Spinnkanne 36 als Flachkanne ausgebildet, die hin und her changiert.

Am Streckwerkseinlauf und am Streckwerksauslauf ist jeweils eine Einrichtung 40 bzw. 41 zur vorzugsweise kontinuierlichen Messung der Faserbandtemperatur angeordnet (in der Figur 1 in unmittelbarer Nähe zu dem jeweiligen Sensor 3 bzw. 30). Die Temperaturmeßwerte bzw. die für die Temperatur charakteristischen Spannungswerte werden der jeweiligen Auswerteeinheit 4 bzw. 31 zugeführt, die hierbei zusätzlich die Funktion einer Kompensationseinheit zur Temperatur-Kompensation der Meßergebnisse des jeweiligen Sensors 3 bzw. 30 übernimmt. Die Ergebnisse der Auswerteeinheiten 4 bzw. 31 sind beispielsweise mittels einer Kreuzkorrelation miteinander korrelierbar. Eine solche Korrelation kann beispielsweise in der Steuereinheit 10 vorgenommen werden, die hierzu entsprechende Rechenleistungen durchführen können muß. Alternativ sind separate Prozessoreinheiten oder eine gemeinsame Prozessoreinheit zur Temperaturkompensation und ggf. auch zur Korrelation der von der Einrichtung 40 und 41 stammenden Signale vorhanden.

Vorzugsweise lassen sich beide Sensoren 3, 30 automatisch reinigen, beispielsweise durch Druckluft aus einer oder mehreren Luftdüsen, die auf den Meßschlitz des jeweiligen Sensors 3, 30 gerichtet sind. Entsprechende Steuereinrichtungen (nicht dargestellt) lösen eine derartige Reinigung aus, vorzugsweise in zeitlichen Abständen und/oder bei Überschreiten eines Grenzwertes der Resonator-Kennwerte (Resonatorgüte) im leeren Zustand und/oder bei Überschreiten von vorgegebenen Schmutz- oder Schmierfilmdicken. Derartige Luftdüsen können auch als Einfädelmittel zum automatischen Einfädeln des oder der zu messenden Faserbänder in den mindestens einen Sensor 3 bzw. 30 dienen.

In einer alternativen, nicht dargestellten Ausführungsform der Erfindung kann die Spinnereivorbereitungsmaschine Einzelantriebe aufweisen, die bevorzugt jeweils eigene Regelkreise besitzen und wobei vorteilhafterweise ein zentraler Rechner eingesetzt wird.

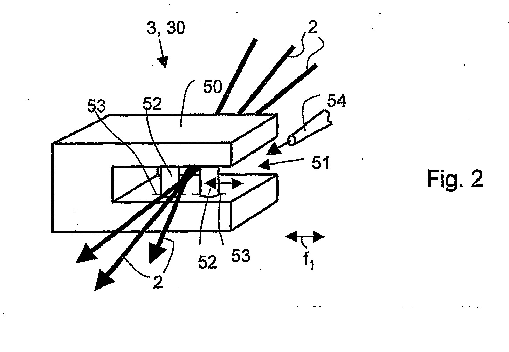

In Figur 2 ist ein offener Mikrowellensensor 3, 30 dargestellt, der aus einem U-förmig gebogenen Resonator 50 besteht, wobei die entsprechende Aussparung als Meßschlitz 51 ausgebildet ist, durch den ein oder mehrere, schematisch als Pfeile angedeutete Faserbänder 2 führbar sind. In dem Meßschlitz 51 ist zu beiden Seiten des oder der Faserbänder 2 je ein Rundstab 52 angeordnet, wobei beide Rundstäbe 52 zusammen als verdichtende Führungselemente dienen. Die Rundstäbe 52 sind auf schematisch angedeuteten Führungen 53 in Bandquerrichtung verschieblich (s. Doppelpfeil) und in der jeweiligen Position vorzugsweise fixierbar angeordnet. Um das oder die Faserbänder 2 in den Meßschlitz 51 und zwischen die beiden Rundstäbe 52 einzuführen, sind ein oder mehrere Luftdüsen 54 vorgesehen, die im wesentlichen in Bandlaufrichtung ausgerichtet sind (in der Figur 2 läuft diese auf den Betrachter zu) und durch dementsprechende Druckluft Faserband in Bandlaufrichtung mitnehmen (s. Pfeil). Des weiteren ist durch den Doppelpfeil f1 angedeutet, daß der gesamte Sensor 3, 30 von einer Meßposition in eine Reinigungsposition und wieder in die Meßposition verfahrbar ausgebildet sein kann.

In den Figuren wurden die einlaufenden Faserbänder 2 und das das Streckwerk 1a verlassende Faserband 2 mit demselben Bezugszeichen bezeichnet. Es ist aus dem Stand der Technik bekannt und deshalb hier nicht näher erläutert, daß die in das Streckwerk 1a einlaufenden Faserbänder 2 das Streckwerk üblicherweise als Faservlies verlassen, welches mittels einer Vliesführungsdüse bzw. Vliesdüse 19 zusammengeführt und mittels eines Bandtrichters zu einem Faserband 2 verdichtet wird, damit dieses in die Spinnkanne 37 abgelegt werden kann. Kombinationen von Vliesdüse und Bandtrichter, wie in Figur 1 schematisch angedeutet, sind möglich.

## Patentansprüche

1. Spinnereivorbereitungsmaschine mit einem regulierten Streckwerk (1 a), welchem mindestens ein Faserband (2) zur Verstreckung vorgelegt wird, wobei das Streckwerk (1a) mindestens ein Eingangswalzenpaar (20) und ein Ausgangswalzenpaar (22) aufweist, mit einer dem Streckwerk (1a) nachgeschalteten Vliesdüse (19), mit einem sich anschließenden Kalanderwalzenpaar (34) zum Verdichten und Abziehen des verstreckten Faserbandes (2), welches anschließend durch einen in einem Drehteller (35) angeordneten Bandkanal in eine Kanne (37) abgelegt wird, **dadurch gekennzeichnet, dass** mindestens ein Mikrowellensensor (30) zur Messung der Faserbanddicke
am Streckwerksauslauf zwischen Vliesdüse (19) und Kalanderwalzenpaar (34) angeordnet ist.

2. Spinnereivorbereitungsmaschine nach Anspruch 1, **dadurch gekennzeichnet, dass** der Mikrowellensensor (3, 30) im Querschnitt eine geschlossene Form aufweist.

3. Spinnereivorbereitungsmaschine nach Anspruch 1, **dadurch gekennzeichnet, dass** der Mikrowellensensor (3, 30) im Querschnitt eine offene Form aufweist, z.B. als gabelförmiger Schlitz.

## Claims

1. Spinning preparation machine with an autoleveling drafting system (1a), to which at least one fibre sliver (2) is presented for drafting, the drafting system (1a) having at least one input roller pair (20) and one output roller pair (22), with a fleece nozzle (19) arranged downstream of the drafting system (1a), with a subsequent calendar roller pair (34) to condense and draw off the drafted fibre sliver (2) which is then deposited into a can (37) via a sliver channel located in a rotary plate (35), **characterized in that** at least one microwave sensor (30) is arranged at the output of the drafting system between the fleece nozzle (19) and the calendar roller pair (34) to measure the fibre sliver thickness.

2. Spinning preparation machine as in claim 1, **characterized in that** the microwave sensor (3, 30) has a closed form in cross section.

3. Spinning preparation machine as in claim 1, **characterized in that** the microwave sensor (3, 30) has an open form in cross-section, e.g. a forked slit.

## Revendications

1. Machine de préparation de filage avec un banc d'étirage régulé (1a), auquel est alimenté au moins un ruban de fibres (2) pour l'étirage, dans lequel le banc d'étirage (1a) comporte au moins une paire de cylindres d'alimentation (20) et une paire de cylindres délivreurs (22), avec une tuyère à non tissé (19) disposée en aval du banc d'étirage (1a), avec une paire de cylindres de calandre (34) lui succédant pour condenser et extraire le ruban de fibres (2) étiré, lequel est ensuite déposé dans un pot (37) à travers un canal à ruban disposé dans un plateau tournant (35), caractérisée en ce qu'au moins un capteur microondes (30) pour la mesure de l'épaisseur du ruban de fibres à la sortie du banc d'étirage est disposé entre la tuyère à non-tissé (19) et la paire de cylindres de calandre (34).

2. Machine de préparation de filage selon la revendication 1, **caractérisée en ce que** le capteur microondes (3, 30) présente en coupe transversale une forme fermée.

3. Machine de préparation de filage selon la revendication 1, caractérisée en ce aue le capteur microondes (3, 30) présente en coupe transversale une forme ouverte, par exemple en fente en forme de fourche.
